# FASCICULE DE BREVET EUROPEEN

(11) **EP 2 864 433 B1**
(45) Date de publication et mention de la délivrance du brevet: **07.06.2023**
(21) Numéro de dépôt: 13737300.7
(22) Date de dépôt: 27.05.2013
(51) Int. Cl.: C09J 7/00

(54) **MATRICE SEQUENCEE MULTIFONCTIONNELLE MONOPOLYMERIQUE EN POLYURETHANNE COULE ET PROCEDE DE FABRICATION**
MULTIFUNKTIONELLE SEQUENZIERTE MONOPOLYMER-GUSS-POLYURETHANMATRIX UND HERSTELLUNGSVERFAHREN
MONOPOLYMER MULTIFUNCTIONAL SEQUENCED CAST POLYURETHANE MATRIX AND PRODUCTION METHOD

(30) Priorité: 26.06.2012 FR 1201794
(43) Date de publication de la demande: 29.04.2015
(73) Titulaire: AB7 Santé, 31450 Deyme (FR)
(72) Inventeur: ALBA, Aurélie, 31450 Noueilles. (FR); VILBERT, Arnaud, 31450 Baziege (FR); CHELLE, René, 31190 Crepiac (FR)
(86) Numéro de dépôt international: PCT/FR2013/000134
(87) Numéro de publication internationale: WO 2014/001653

(56) Documents cités:
- EP-A2- 0 033 615
- EP-A2- 0 212 681
- EP-A2- 2 324 803
- WO-A2-00/59483
- US-A- 5 662 926

## Description

La présente invention concerne une matrice séquencée multifonctionnelle monopolymérique en polyuréthanne coulé, remplissant concomitamment les fonctions de support, ou de support-barrière, de réservoir d'actif(s) et d'adhésif, lesdites fonctions étant obtenues grâce à un procédé de coulée séquentielle du polymère formulé suivant des phases induisant les caractéristiques correspondant aux différentes fonctions énoncées pour chaque phase pour constituer ladite matrice formant un dispositif de distribution d'actifs.

Les dispositifs matriciels de distribution d'actifs tels les patchs, les emplâtres et autres, divulgués par exemple par le brevet EP0341202 et la demande américaine US2011038904, sont généralement de structure stratifiée organisée principalement en trois couches essentielles :
- une couche support ou de recouvrement en polymère imperméable à l'actif, généralement en Polyéthylène téréphtalate (PET), en Polypropylène (PP), en Chlorure de polyvinyle (PVC), en Polybutilène téréphtalate (PBT), en Polyéthylène (PE), en Copolymère d'éthylène et d'acétate de vinyle (EVA), ou encore en polyuréthane, laquelle couche support peut aussi faire office de barrière aux actifs ;
- une couche réservoir dans laquelle sont emmagasinés les actifs, qui peut être en gel de polyuréthane (PU) pour bénéficier de l'avantage de la régularité de relargage des actifs apportée par ce polymère, en gélatine, en polymère d'alcool polyvinylique, en méthylcellulose, ou en un des copolymères à blocs, ou encore en d'autres polymères réticulés pouvant emmagasiner une substance active pour la relarguer lentement. Les actifs sont incorporés par fusion du polymère (hot melt) ou par solubilisation dudit polymère avec un solvant organique comme décrit dans les brevets EP1169025 et EP0416842 ;
- une couche auto-adhésive constituée par des polymères ayant des propriétés adhésives comme les polysiloxanes, les polyacrylates, les silicones, les polymères vinyliques, l'acétate de vinyle, le copolymère d'éthylène et d'acétate de vinyle, les polyisobutylènes, les polyuréthannes, les gommes naturelles ou les caoutchoucs naturels et synthétiques, diversement décrits par les brevets EP1169025, EP0416842, EP0836506, EP0563507 et EP2324859 pour ne citer que ceux-là, lesquelles substances permettant de fixer le dispositif sur une surface donnée ou sur la peau.

La réalisation d'un tel dispositif est techniquement complexe. En effet, il présente l'inconvénient d'être pratiquement difficile à réaliser, car il faut tenir compte de la compatibilité des différents matériaux utilisés, ainsi que de la multiplicité des opérations technologiques à exécuter. Le bon fonctionnement des dispositifs qui en sont issus dépend des propriétés de chacune des couches des différents polymères dont dépendent à leur tour les fonctions qui leur sont attribuées [EP 2 191 810]. Chaque couche est préparée à part, selon un procédé particulier, ce qui multiplie les opérations et les contraintes technologiques, y compris en outillages utilisés.

Le support, film en PET, PP, PVC, PBT, PE ou encore EVA requiert une extrusion à chaud, consommatrice d'énergie, ou une solubilisation dans un solvant qu'il faut recycler ou éliminer en tenant compte de l'impact environnemental. Dans le cas où le film est en polyuréthane, nous avons les exemples de la demande de brevet japonais WO 2011136330 qui décrit un film extrudé de 1 à 10 µm d'épaisseur, la demande de brevet européen EP 2 324 803 A2 qui utilise un film obtenu par fusion soufflage, tandis que la demande de brevet EP0212681 A2 divulgue un film obtenu par fusion de polyuréthane par radiation actinique.

Dans le cas où le réservoir est du polyuréthane, il est généralement conçu individuellement avant d'être monté dans un système multicouche. Ainsi le brevet EP1169025 décrit un dispositif comprenant une couche réservoir élaborée à partir de granulés de polyuréthanne fondus pour être mélangé entre 40°C et 90°C avec le médicament, du fentanyl. Le brevet EP1634566 divulgue un réservoir dont la structure même du polyuréthanne est modifiée par greffage de segments d'oxyde d'éthylène hydrophile ainsi que de segments d'oxyde de propylène hydrophobes pour y incorporer de la vitamine C. Là encore, une dépense d'énergie importante s'impose. En plus, le traitement du polymère à chaud est une source de détérioration ou de dénaturation des actifs thermolabiles.

Dans le cas où la couche adhésive est en polyuréthanne, elle est conçue grâce à l'ajout de silicone ou de copolymère de méthacrylate d'alkyle comme décrit par le brevet EP2191810. C'est une opération supplémentaire qui exige une certaine rigueur dans le dosage des différents matériaux utilisés.

Pour minimiser la difficulté inhérente au système multicouche, la tendance dans la pratique est à la réduction du nombre de couches du dispositif stratifié. Ainsi on observe la bifonctionnalisation de la couche réservoir en couche réservoir auto-adhésive. Pour ce faire, le polyuréthane est associé à des polymères acryliques [EP2001424, EP2431437, EP1875898, EP2412390, EP0341202, US6727401]. Le brevet EP1263830 décrit une matrice adhésive en un PU obtenu à partir d'un système aqueux, en utilisant deux polyols de poids moléculaire PM < 2000 pour l'un et PM > 2000 pour l'autre. La somme des deux polyols est en excès par rapport à l'isocyanate pour obtenir un polyuréthanne dit sous-indexé. Mais la matrice adhésive obtenue n'est applicable sur un substrat de dispersion qu'en chauffant pendant 10 minutes à 70°C.

La couche auto-adhésive en polyuréthanne obtenue en général comme décrit est bien collante sur les deux faces, ce qui oblige, lorsque l'on désire n'avoir qu'une face collante, de couvrir l'autre face de ladite couche auto-adhésive avec un film barrière en polymère d'alcool polyvinylique (PVA) ou en copolymère d'éthylène et d'acétate de vinyle (EVA), offrant une face non adhésive comme décrit par la demande de brevet EP 1 634 566 A1.

Il est connu de l'homme du métier que l'effet adhésif d'un PU peut être acquis en jouant sur la stoechiométrie de la réaction polymérisante entre l'isocyanate et le(s) polyol(s), exprimé ici en rapport OH/NCO. Lorsqu'on a l'isocyanate en excès, la stoechiométrie est inférieure à 1. On peut obtenir un effet adhésif évolutif par réaction des radicaux NCO libres. Par contre, lorsqu'on a les radicaux OH du (des) polyol(s) en excès, la stoechiométrie est supérieure à 1. On obtient un effet adhésif stable et efficace.

Il existe bien un besoin de fournir une nouvelle conception technique permettant d'obtenir une matrice multifonctionnelle monopolymérique en polyuréthanne coulé en remplacement des structures stratifiées complexes dont sont élaborés les dispositifs auto-adhésifs distributeurs d'actifs.

Pour mieux comprendre l'invention, on entend par « matrice séquencée multifonctionnelle mopolymérique en polyuréthanne coulé » une matrice obtenue en mélangeant un complexe polyol liquide de composition variable avec un isocyanate liquide pour obtenir une solution de phase liquide, puis en coulant ladite solution de phase séquentiellement dans un moule pour obtenir une succession de séquences, en veillant à ce que la solution de phase coulée sur la séquence précédente le soit avant la fin de polymérisation de ladite séquence précédente pour obtenir une copolymérisation à l'interface des deux séquences.

On entend par « complexe polyol » la solution du (des) polyol(s) avec les différents additifs techniques (catalyseur, allongeur, plastifiant, débullant, anti UV, etc.) et le cas échéant le(s) actif(s). Sa composition varie en concordance de la fonction et des caractéristiques souhaitées pour la séquence à polymériser.

On entend par « *solution de phase* » le mélange du complexe polyol liquide avec l'isocyanate liquide.

On entend par «*séquence* » la solution de phase en polymérisation dans le moule. La « *séquence* » est déterminée à la fois par la composition du complexe polyol et la stoechiométrie de la solution de phase.

On entend par « *profil de la matrice* » l'organisation selon l'ordre de succession des différentes séquences composant la matrice.

On entend par « pot life » le niveau de polymérisation caractérisé par un étirement en fil du gel qui commence à se former de la solution de phase. Le pot life est déterminé expérimentalement et exprimé en unités de temps, généralement en secondes.

On entend par « temps de démoulage » le niveau de polymérisation à partir duquel le démoulage de la matrice polymérique devient possible sans endommager le dispositif moulé. Le temps de démoulage est déterminé expérimentalement et exprimé en unités de temps.

Un des objets de l'invention est de réaliser une matrice séquencée multifonctionnelle monopolymérique en polyuréthanne coulé, remplissant concomitamment les fonctions de support ou de support-barrière, de réservoir d'actif(s) et d'adhésif selon un arrangement voulu des séquences fonctionnalisées définissant un profil particulier de ladite matrice.

La demanderesse a découvert de manière surprenante qu'une telle matrice pouvait être obtenue de manière simple et efficace, en coulant séquentiellement dans un moule successivement différentes solutions de phase d'isocyanate/complexe polyol avec alternance d'objectivation des solutions de phase selon un ordre convenu et dans le respect du stade d'évolution de la polymérisation (après le pot life et avant le temps de démoulage) de la séquence précédant celle en cours de coulage, pour clôturer par le coulage de la dernière solution de phase que l'on va laisser aller jusqu'à son temps de démoulage et achever ainsi la polymérisation de l'ensemble de la matrice.

Selon un aspect de l'invention, la matrice séquencée multifonctionnelle monopolymérique en polyuréthanne coulé présente une unicité ou continuité structurelle assurée par l'état de copolymérisation à l'interface des séquences fonctionnelles successives.

La matrice selon l'invention est chargée dans ses séquences « réservoir » en actifs d'origine naturelle ou de synthèse, pénétrants transdermiques ou volatils. Lesdits actifs utilisés selon l'invention sont des substances médicamenteuses et thérapeutiques, des substances cosmétiques, des substances de bien-être, des substances phytosanitaires, des répulsifs, des attractifs, des phéromones, des biocides, des parfums ou des désodorisants.

Selon le mode de réalisation de l'invention, le(s) actif(s) représente(nt) entre 0,01% et 40% en poids de la matrice séquencée multifonctionnelle monopolymérique en polyuréthanne coulé.

En effet, selon l'invention, la même matrice peut contenir plusieurs actifs de nature différente, appartenant à des domaines d'activité différents et pouvant agir soit dans la même direction, soit dans des directions opposées. Par exemple un parfum apaisant, du domaine d'activité atmosphérique, peut être associé dans la même matrice avec un actif médicamenteux antidouleur, du domaine d'activité transdermique. Les deux actifs étant chargés dans des séquences « réservoir » différentes.

De même, selon un mode de réalisation de l'invention, deux ou plusieurs actifs appartenant au même domaine d'activité, chargés dans des séquences « réservoir » différentes dans la même matrice, peuvent agir unidirectionnellement mais à vitesses et débits différents.

Ladite matrice selon l'invention peut servir directement à la mise en forme de dispositifs variés qui adhèrent à la peau ou toute autre surface, tout en délivrant une ou plusieurs substances actives de manière contrôlée.

Lesdits dispositifs selon l'invention peuvent être des patchs, des patchs avec plusieurs actifs à actions unidirectionnelles ou à directions opposées (exemple vers la peau et vers l'atmosphère), des emplâtres changeables collés sur support textile (harnais, bandages, sous vêtements), des talonnettes actives et des coussinets actifs pour chaussures, des plaquettes à phéromone pour le contrôle des insectes, des plaquettes auto-adhésives attractives insecticides, ou d'autres dispositifs équivalents en terme de structure et de fonction.

A cet effet, la matrice séquencée multifonctionnelle monopolymérique en polyuréthanne coulé selon l'invention a une épaisseur qui évolue d'une pellicule de 10 à 100 microns d'épaisseur à un bloc polymère de plusieurs centimètres d'épaisseur dans lequel chaque séquence peut avoir une épaisseur différente. L'épaisseur de la séquence réservoir par exemple va varier en fonction du volume d'actif(s) à emmagasiner et de la durée de l'activité souhaitée.

Un autre objet de l'invention est de fournir un procédé d'élaboration de la matrice séquencée multifonctionnelle monopolymérique en polyuréthanne coulé.

Ainsi, selon l'invention, le procédé de préparation d'une matrice séquencée multifonctionnelle monopolymérique en polyuréthanne coulé à température ambiante, structurée concomitamment en séquence support ou support-barrière, séquence réservoir d'actif(s) et séquence adhésive, ladite matrice présentant une unicité ou continuité structurelle assurée par la copolymérisation à l'interface desdites séquences fonctionnelles successives de fonctionnalités différentes, lesdites séquences étant formées par des solutions de phase constituées par le mélange d'un complexe polyol liquide variable avec un isocyanate liquide, comprend les étapes suivantes :
a) préparation du complexe polyol 1 qui consiste à mélanger la solution de catalyseur, les différents additifs et le cas échéant le(s) actif(s) correspondant à la fonction de la future séquence 1 et le(s) polyol(s) ;
b) préparation de la solution de phase 1 qui consiste à mélanger le complexe polyol 1 obtenu en a)- avec l'isocyanate selon la stoechiométrie correspondant à la fonction de la future séquence 1 ;
c) coulage de ladite solution de phase 1 dans le moule pour l'obtention de la séquence 1 en conformité de l'épaisseur souhaitée de ladite séquence 1 ;
d) préparation du complexe polyol 2 qui consiste à mélanger la solution de catalyseur, les différents additifs et le cas échéant le(s) actif(s) correspondant à la fonction de la future séquence 2 et le(s) polyol(s) ;
e) préparation de la solution de phase 2 qui consiste à mélanger le complexe polyol 2 obtenue en d)- avec l'isocyanate selon la stoechiométrie correspondant à la fonction de la future séquence 2 ;
f) coulage de ladite solution de phase 2 dans le moule pour l'obtention de la séquence 2, en conformité de l'épaisseur souhaitée de ladite séquence 2, sur la séquence 1 ayant dépassé le pot life mais n'ayant pas encore atteint le temps de démoulage, pour permettre une copolymérisation à l'interface des deux séquences ;
g) répéter les étapes a)- à f)- pour obtenir une matrice multifonctionnelle à « n » séquences, la dernière et/ou la première selon le profil choisi de la matrice étant une séquence au moins adhésive dans laquelle la solution de phase du complexe polyol avec l'isocyanate est de stoechiométrie supérieure à 1 ;
h) laisser s'achever la polymérisation de la dernière séquence jusqu'à son temps de démoulage et donc de l'ensemble de la matrice,
i) .démouler la matrice séquencée multifonctionnelle monopolymérique formée,
j) procéder à l'emballage de ladite matrice dans une poche scellée, étanche et imperméable

Selon le procédé de l'invention, la surface de la séquence en pleine polymérisation offre des radicaux réactifs encore libres avec lesquels réagissent immédiatement les radicaux apportés par la solution de phase en cours de coulage pour engendrer une copolymérisation sur cette interface lorsque ladite solution de phase polymérise à son tour pour donner une séquence. Ladite séquence va ainsi former un ensemble avec la séquence précédente, sans pour autant qu'il y ait interpénétration des deux séquences. La continuité et donc l'unicité de la matrice séquencée multifonctionnelle monopolymérique en polyuréthanne coulé est conditionnée par le phénomène de copolymérisation des séquences fonctionnelles successives à leurs interfaces de contact en cours de polymérisation.

Selon un mode de réalisation de l'invention, la séquence *Support* peut être imperméabilisée pour devenir un *Support-Barrière* par rapport aux actifs par ajout de résine(s) à faible poids moléculaire et/ou des charges inertes.

Plus précisément, selon l'invention, le procédé d'élaboration de la matrice séquencée multifonctionnelle monopolymérique en polyuréthanne coulé est réalisable avec les principaux matériaux réactifs de polymérisation qui sont :
a), l'isocyanate est choisi parmi les isocyanates avec au minimum 2 fonctionnalités, de structure aromatique ou aliphatique ;
b). le(s) polyol(s) choisis parmi ceux ayant au minimum 2 fonctionnalités hydroxyle, préférentiellement à chaîne longue et selon les cas à chaîne courte, à base de polyesters, de polyéthers, de polythioéthers, de polyacétals, de polycarbonates, de polyesteramides, d'huiles végétales naturellement hydroxylées ou modifiées ou le mélange de ceux-ci..

Les additifs de formulation du complexe polyol suivant l'invention sont choisis selon le type de fonction que va remplir chaque séquence et les caractéristiques souhaitées globalement du dispositif à former que sont :
**a).** le catalyseur choisi parmi les solutions d'organométalliques, de préférence de bismuth, ou les solutions d'amines tertiaires;
**b).** les débullants choisis parmi les polymères de polysiloxanes, les polymères de methylalkylpolysiloxanes ou un mélange de ceux-ci ;
**c).** le plastifiant choisi parmi les solutions de benzoate d'ester, les alkyls phosphate ainsi que ceux connus de l'homme de métier ;
**d).** l'imperméabilisant choisi parmi les résines de faible poids moléculaire ou les charges inertes de préférence de nature siliceuse ;
**e).** les anti UV choisis parmi ceux connus de l'homme de métier et les autres stabilisants choisis parmi les antioxydants phénoliques de type BHT (Butylhydroxytoluène) ou BHA (Butylhydroxyanisol), les antioxydants redox de type HALS (Hindered Amine Light Stabilizer) à motif pipéridine, les antioxydants de type phosphite, les antioxydants de type formamidine, les antioxydants de type benzotriazole, les antioxydants de type benzophénone ou un mélange de ceux-ci ;
**f).** les colorants des polyuréthannes connus de l'homme de métier.

Selon un mode de réalisation de l'invention, la matrice séquencée multifonctionnelle mopolymérique en polyuréthane coulé présenter plusieurs possibilités de combinaisons de séquences fonctionnelles pour obtenir des profils qui permettent une grande maîtrise du contrôle de libération des actifs, particulièrement dans les cas suivants :
**A).** *Support* / *Réservoir* / *Adhésive* pour permettre à l'actif se trouvant dans la séquence réservoir à traverser la séquence adhésive pour une délivrance transdermique par exemple ;
**B).** *Réservoir 1* / *Support-Barrière* / *Réservoir 2-Adhésive* pour deux actifs différents : celui du *Réservoir 1* est destiné à une libération par volatilité dans l'atmosphère, tandis que celui du *Réservoir 2* est destiné à une délivrance par voie transdermique ;
**C).** *Support* / *Réservoir-Adhésive* pour un actif à libération rapide par voie transdermique ;
**D).** *Réservoir* / *Support-Barrière* / *Adhésive* pour un actif volatil à libération dans l'atmosphère et qui ne doit pas être au contact de la peau ;
**E).** *Support* / *Réservoir 1* / *Réservoir 2* / *Adhésive* pour deux actifs différents se trouvant respectivement dans les séquences Réservoir 1 et Réservoir 2 à vitesse et débit de délivrance différés par voie transdermique ;
**F).** *Support* / *Réservoir 1* / *Réservoir 2-Adhésive* pour deux actifs différents se trouvant respectivement dans les séquences Réservoir 1 et Réservoir 2-Adhésive à vitesse et débit de libération différenciés par voie transdermique, mais avec une grande accélération de la libération de l'actif du *Réservoir 2-Adhésive.*

Le procédé selon l'invention apporte les avantages technologiques suivants :
**a).** simplification de la mise en oeuvre des différents profils de ladite matrice ;
**b).** grandes possibilités d'automatisation poussée de la production de dispositifs ;
**c).** grandes possibilités de formes de dispositifs et donc d'utilisations ;
**d).** grandes possibilités de réglage des fonctionnalités mécaniques et actives ;
**e).** préservation de la qualité des actifs du fait du traitement à température ambiante ;
**f).** absence de solvants pour une préservation de la nature et une économie d'énergie de leur élimination ;
**g).** large gamme d'actifs utilisables grâce aux grandes capacités d'emmagasinage du polyuréthanne coulé tant pour les substances lipophiles que pour les substances hydrophiles, d'origine naturelle ou de synthèse.

Une machine de coulée permettant de mettre en oeuvre le procédé selon l'invention est constituée au minimum par deux réservoirs, un pour le complexe polyol et l'autre pour l'isocyanate, équipés de dispositifs de réglage de débit et d'un système de poussée des liquides sous basse pression vers un mélangeur permettant de distribuer la solution de phase liquide dans le moule de polymérisation pour le formage du dispositif voulu.

Selon un mode directement industrialisable de l'invention, la production de dispositifs en matrice séquencée multifonctionnelle monopolymérique en polyuréthanne coulé est réalisée dans une machine de coulée, dont la configuration (capacité de production, nombre de réservoirs de liquides, débit d'écoulement des liquides, pression de poussée des liquides, ordre de poussée des liquides selon les réservoirs, profil de la turbine, nature du ou des moules) est adaptée par l'homme de métier, et équipée d'un dispositif de supervision électronique.

### EXEMPLES

Les exemples qui suivent servent à illustrer l'invention, sans être exhaustifs, sans en limiter la portée qui s'étend à des dispositifs équivalents en terme de structure et de fonction.

### Exemple 1 : Patch anti-douleur auto-adhésif en matrice ayant un profil « Support/Réservoir-Adhésive » :

Pour l'élaboration d'une matrice active monopolymérique apportant une fonction auto-adhésive sur une seule face d'un patch, de profil matriciel organisé en succession de deux séquences : une séquence support et une séquence réservoir adhésive chargée en actif anti-douleur.

On dispose pour cela de :
- un polyol à chaîne longue à base d'éther, plus précisément un polypropylène glycol (PPG) de PM=4800 et de fonctionnalité 3. Ce polyol est produit par REPSOL et commercialisé sous la marque ALCUPOL^{®} C-3531 ;
- un diisocyanate aromatique de type méthylène diphényle 4,4-diisocyanate (MDI), de PM=344 et présentant un taux de NCO=24,4% et commercialisé par HUNTSMAN sous la marque SUPRASEC^{®} 2029 ;
- un débullant qui est une solution de polymères et de polysiloxanes, produit par BYK CHEMIE et commercialisé sous la marque BYK 088 ;
- un plastifiant qui est un mélange d'esters de benzoate commercialisé par VELSICOL sous la marque BENZOFLEX^{®} 9-88 SG ;
- un agent catalyseur qui est un complexe néodécanoate de bismuth commercialisé par SHEPHERD sous la marque BICAT^{®} 8124M ;
- un mélange d'huiles essentielles à action anti-douleur commercialisées par INTERAXION.

On dispose du matériel suivant :
- un réacteur cylindrique ouvert de 1 litre ;
- un agitateur métallique muni d'un axe de 25 cm de longueur, se terminant par un disque de 6 cm de diamètre. L'agitateur est actionné par un moteur à variation de vitesse ;
- un moule en polypropylène (PP) dont l'empreinte est un parallélépipède ouvert de 7 cm x 5 cm de côtés et 5 mm de profondeur.

Mode opératoire : Toutes les opérations sont réalisées à température ambiante.

### A)- Préparation de la solution de phase « Support » de stoechiométrie OH/NCO = 1

∘ On prépare le catalyseur en mettant en solution 5% du BICAT^{®} 8124M dans le plastifiant.
∘ On introduit successivement dans le réacteur :
   ▪ 3,06 parties en poids de la solution de catalyseur ;
   ▪ 1,24 parties en poids de débullant et
   ▪ 86,42 parties en poids de polyol à chaîne longue.
∘ On agite le tout à 1300 trs/mn pendant 30 secondes. On obtient le complexe polyol ;
∘ On ajoute dans ledit complexe polyol 9,27 parties en poids d'isocyanate selon une stoechiométrie OH/NCO=1, et on agite à 1300 trs/mn pendant 30 secondes pour obtenir la solution de phase ;
∘ On coule ladite solution de phase obtenue dans le moule sur une épaisseur de 1 mm, et on laisse la réaction de polymérisation se faire pour constituer la séquence « support ».

### B)- Préparation de la solution de phase « Réservoir-Adhésive » de stoechiométrie OH/NCO = 1,6

∘ Parallèlement à l'opération A), on modifie le complexe polyol en associant dans le réacteur :
   ▪ 2,55 parties en poids de solution de catalyseur ;
   ▪ 1,03 parties en poids de débullant ;
   ▪ 16,86 parties en poids du mélange d'huiles essentielles à action anti-douleur, dit l'actif et
   ▪ 74,56 parties en poids de polyol à chaîne longue de l'opération A).
∘ On agite à 1300 trs/mn pendant 30 secondes. On obtient le complexe polyol chargé d'actif anti-douleur ;
∘ On ajoute dans ledit complexe polyol chargé obtenu 4,99 parties en poids d'isocyanate selon une stoechiométrie OH/NCO=1,6 et on agite à 1300 trs/mn pendant 30 secondes. On obtient la solution de phase chargée d'actif ;
∘ On coule sur une épaisseur de 1 mm ladite solution de phase sur la séquence « Support » qui a dépassé le « pot life » mais n'a pas atteint le temps de démoulage et donc encore en pleine polymérisation ;
∘ On laisse se continuer la polymérisation pour constituer la séquence « Réservoir-Adhésive » ;
∘ On démoule le patch ainsi obtenu lorsque le temps de démoulage de la dernière séquence « Réservoir-Adhésive » est atteint.

Le patch obtenu, d'une épaisseur de 2 mm, est très souple et adhésif sur la face de la séquence « Réservoir-Adhésive » et non collant sur la face de la séquence « Support ». Ledit patch auto-adhésif est destiné au traitement de la douleur musculaire. Il est collé directement sur la peau.

### Exemple 2 : Emplâtre apaisant auto-adhésif en matrice ayant un profil « Réservoir-Support ladhésive »

Cet exemple illustre l'élaboration d'une matrice monopolymérique active apportant une fonction auto-adhésive d'un emplâtre sur une seule face, selon un profil matriciel organisé en succession de deux séquences : une séquence réservoir support chargée d'actif apaisant et une séquence adhésive.
- On associe à l'isocyanate et au polyol à chaîne longue de l'exemple 1 un autre polyol à chaîne longue à base d'éther (PPG) de PM=2000 et de fonctionnalité de 2. Ce polyol à chaîne longue est également produit par REPSOL et commercialisé sous la marque ALCUPOL^{®} D-2021.
- On dispose également d'un stabilisant comprenant un mélange de formamidines et commercialisé par ZIKO sous la marque ZICA-CUV^{®}, ainsi que d'un parfum apaisant commercialisé par ROBERTET sous la marque ELISIA^{®}+.

On dispose du même matériel que celui utilisé dans l'exemple 1 et d'un moule dont l'empreinte est un parallélépipède ouvert de 14 cm x 10 cm de côtés et 5 mm de profondeur.

Mode opératoire : On procède selon un mode opératoire analogue à celui de l'exemple 1 et la solution de catalyseur est préparée comme dans l'exemple 1.

### A)- Préparation de la solution de phase « Réservoir-Support » de stoechiométrie OH/NCO = 1

∘ On introduit successivement dans le réacteur :
   ▪ 2,97 parties en poids de solution de catalyseur ;
   ▪ 0,15 partie en poids de stabilisant ;
   ▪ 1,02 partie en poids de débullant ;
   ▪ 3,06 parties en poids de parfum à action apaisante, dit l'actif ;
   ▪ 49,84 parties en poids de polyol à chaîne longue (ALCUPOL^{®} C-3135) et
   ▪ 31,09 parties en poids de polyol à chaîne longue (ALCUPOL^{®} D-2021)
∘ On agite le tout à 1300 trs/mn pendant 30 secondes. On obtient le complexe polyol chargé d'actif apaisant ;
∘ On ajoute dans ledit complexe polyol ainsi obtenu 11,87 parties en poids d'isocyanate selon une stoechiométrie OH/NCO = 1, et on agite à 1300 trs/mn pendant 30 secondes. On obtient la solution de phase chargée d'actif ;
∘ On coule ladite solution de phase obtenue dans le moule sur une épaisseur de 1 mm, et on laisse la réaction de polymérisation se faire pour constituer la séquence « Réservoir-Support ».

### B)- Préparation de la solution de phase « Adhésive » de stoechiométrie OH/NCO=1,4

∘ Parallèlement à l'opération A), on modifie le complexe polyol en associant dans le réacteur :
   ▪ 3,06 parties en poids de solution de catalyseur ;
   ▪ 0,16 partie en poids de stabilisant ;
   ▪ 1,06 partie en poids de débullant ;
   ▪ 53,54 parties en poids de polyol à chaîne longue de l'opération A) (ALCUPOL^{®} C-3531) et
   ▪ 33,83 parties en poids de polyol à chaîne longue de l'opération A) (ALCUPOL^{®} D-2021).
∘ On agite à 1300 trs/mn pendant 30 secondes. On obtient le complexe polyol ;
o On ajoute dans ledit complexe polyol obtenu 8,25 parties en poids d'isocyanate selon une stoechiométrie OH/NCO = 1,4 et on agite à 1300 trs/mn pendant 30 secondes. On obtient la solution de phase ;
∘ On coule sur une épaisseur de 1 mm, ladite solution de phase obtenue sur la séquence « Réservoir-Support » qui a dépassé le pot life mais qui n'a pas encore atteint le temps de démoulage ;
∘ On laisse se continuer la polymérisation pour constituer la séquence « Adhésive » ;
o On démoule l'emplâtre ainsi obtenu lorsque le temps de démoulage de ladite séquence « Adhésive » est atteint.

L'emplâtre apaisant obtenu est très souple et présente une épaisseur de 2 mm. L'emplâtre est collé par sa face adhésive sur un harnais en textile. Il est destiné au traitement de l'anxiété chez le chien adulte grâce au harnais fixé autour de la taille du chien, emplâtre contre le pelage.

### Exemple 3 : Coussinet parfumant auto-adhésif pour la pointe de chaussure en matrice ayant un profil « Réservoir/Support-Barrière/ Adhésive »

Cet exemple illustre l'élaboration d'une matrice active monopolymérique apportant une fonction auto-adhésive d'un coussinet pour chaussure sur une seule face, selon un profil matriciel organisé en succession de trois séquences : une séquence réservoir chargée d'actif, une séquence support barrière et une séquence adhésive.
- On associe à l'isocyanate et au polyol à chaîne longue de l'exemple 1 un autre polyol à chaîne longue ALCUPOL^{®} D-2021.
- On dispose également de :
   ∘ un débullant BYK 088,
   ∘ un stabilisant ZICA-CUV^{®},
   o un parfum commercialisé par CREATIONS & PARFUM sous la marque BSG^{®}51774.
- On dispose du même matériel que celui utilisé dans l'exemple 1 et d'un moule métallique dont l'empreinte est un coussinet pour la pointe des pieds de 9,5 cm de longueur sur 7 cm de largeur et 3 mm de profondeur.

Mode opératoire : On procède selon un mode opératoire analogue à celui de l'exemple 1 et la solution de catalyseur est préparée comme dans l'exemple 1.

### A)- Préparation de la solution de phase « Réservoir » de stoechiométrie OH/NCO = 1

∘ On introduit successivement dans le réacteur :
   ▪ 2,75 parties en poids de solution de catalyseur ;
   ▪ 0,13 partie en poids de stabilisant ;
   ▪ 0,91 partie en poids de débullant ;
   ▪ 4,58 parties en poids de parfum, dit l'actif et
   ▪ 82,76 parties en poids de polyol à chaîne longue (ALCUPOL^{®} C-3531).
∘ On agite le tout à 1300 trs/mn pendant 30 secondes. On obtient le complexe polyol chargé d'actif ;
∘ On ajoute dans ledit complexe polyol obtenu 8,87 parties en poids d'isocyanate selon une stoechiométrie NCO/OH=1, et on agite à 1300 trs/mn pendant 30 secondes. On obtient la solution de phase chargée d'actif ;
∘ On coule ladite solution de phase obtenue dans le moule sur une épaisseur de 1 mm, et on laisse la réaction de polymérisation se faire pour constituer la séquence « Réservoir ».

### B)- Préparation de la solution de phase « Support-Barrière » de stoechiométrie OH/NCO = 1

∘ Parallèlement à l'opération A)-, on modifie le complexe polyol en associant dans le réacteur :
   ▪ 1,95 parties en poids de la solution de catalyseur ;
   ▪ 0,49 partie en poids de débullant et
   ▪ 59,65 parties en poids de polyol à chaîne longue (ALCUPOL^{®} C-3531)
∘ On agite le tout à 1300 trs/mn pendant 30 secondes. On obtient le complexe polyol ;
∘ On ajoute dans ledit complexe polyol 37,92 parties en poids d'isocyanate selon une stoechiométrie OH/NCO=1 et on agite à 1300 trs/mn pendant 30 secondes. On obtient la solution de phase ;
∘ On coule sur une épaisseur de 1 mm ladite solution de phase obtenue sur la séquence « Réservoir » qui a dépassé le pot life mais qui n'a pas encore atteint le temps de démoulage et on laisse la réaction de polymérisation se faire pour constituer la séquence « Support-Barrière ».

### C)- Préparation de la solution de phase « Adhésive » de stoechiométrie OH/NCO=1,4

∘ Parallèlement à l'opération B), on modifie le complexe polyol en associant dans le réacteur :
   ▪ 3,06 parties en poids de solution de catalyseur ;
   ▪ 0,16 partie en poids de stabilisant ;
   ▪ 1,06 partie en poids de débullant ;
   ▪ 53,54 parties en poids de polyol à chaîne longue de l'opération A) (ALCUPOL^{®} C-3531) et
   ▪ 33,83 parties en poids de polyol à chaîne longue de l'opération A) (ALCUPOL^{®} D-2021).
∘ On agite à 1300 trs/mn pendant 30 secondes. On obtient le complexe polyol ;
∘ On ajoute dans ledit complexe polyol obtenu 8,25 parties en poids d'isocyanate selon une stoechiométrie OH/NCO=1,4 et on agite à 1300 trs/mn pendant 30 secondes. On obtient la solution de phase ;
∘ On coule sur une épaisseur de 1 mm, ladite solution de phase obtenue sur la séquence « Support Barrière » qui a dépassé le pot life mais qui n'a pas encore atteint le temps de démoulage ;
∘ On laisse se continuer la polymérisation pour constituer la séquence « Adhésive » ;
∘ On démoule le coussinet ainsi obtenu lorsque le temps de démoulage de ladite séquence « Adhésive » est atteint.

Le coussinet parfumant obtenu d'une épaisseur de 3 mm, est souple et auto-adhésif. Ledit coussinet colle sur la semelle de la chaussure sur la face de la séquence adhésive. Il permet également de parfumer l'intérieur de la chaussure. De par les propriétés mécaniques de la matrice (souplesse, élasticité et amortissement), le coussinet apporte du confort au porteur.

### Exemple 4 : Patch de bien-être et amincissant auto-adhésif en matrice ayant un profil «RéservoirlSupport-BarrièrelAdhésive-Réservoir »

Cet exemple illustre l'élaboration d'une matrice monopolymérique à double activité dont une activité à effet atmosphérique et une activité à effet transdermique, apportée par un patch auto-adhésif sur une seule face. Ledit patch présente un profil matriciel organisé en succession de trois séquences : une séquence réservoir chargée d'actif volatil, une séquence support barrière et une séquence réservoir adhésive chargée d'actif transdermique.

On associe à l'isocyanate de l'exemple 1 un polyol à chaîne courte à base d'huile végétale, de PM = 700, de fonctionnalité de 2, produit par ALBERDINGK BOLEY et commercialisé sous la marque ALBODRY^{®}.

On dispose de :
- un polyol à chaîne courte à base d'éther, plus précisément de type (PPG) de PM = 400 et de fonctionnalité de 2. Ce polyol est produit par DOW CHEMICAL et commercialisé sous la marque VORANOL P400.
- un polyol à chaîne longue à base de polycarbonate, de PM=1000 et de fonctionnalité de 2. Ce produit est produit par UBE CHEMICAL et commercialisé sous la marque ETERNACOLL^{®} PH100.
- un allongeur de chaîne de PM = 90 et de fonctionnalité de 2 connu sous le nom chimique de 1,4- Butanediol
- un débullant BYK^{®} 066.
- un parfum commercialisé par CREATIONS & PARFUM sous la marque BSG^{®}51774.
- une préparation lipophile de caféine anhydre à action amincissante formulée par AB7 INDUSTRIES S.A.

On dispose du même matériel que celui utilisé dans l'exemple 1.

Mode opératoire : On procède selon un mode opératoire analogue à celui de l'exemple 1 et la solution de catalyseur est préparée comme dans l'exemple 1.

### A)- Préparation de la solution de phase « Réservoir» de stoechiométrie OH/NCO = 1

∘ On introduit successivement dans le réacteur :
   ▪ 1,57 parties en poids de la solution de catalyseur ;
   ▪ 0,86 partie en poids d'allongeur de chaîne ;
   ▪ 0,39 partie en poids de débullant ;
   ▪ 19,61 parties en poids de parfum, dit l'actif et
   ▪ 49,87 parties en poids de polyol à chaîne courte (ALBODRY^{®})
∘ On agite le tout à 1300 trs/mn pendant 30 secondes. On obtient le complexe polyol chargé d'actif ;
∘ On ajoute dans ledit complexe polyol 27,71 parties en poids d'isocyanate selon une stoechiométrie OH/NCO = 1, et on agite à 1300 trs/mn pendant 30 secondes. On obtient la solution de phase chargée d'actif parfum;
∘ On coule ladite solution de phase obtenue dans le moule sur une épaisseur de 1 mm, et on laisse la réaction de polymérisation se faire pour constituer la séquence « Réservoir ».

### B)- Préparation de la solution de phase « Support-Barrière » de stoechiométrie OH/NCO = 1

∘ Parallèlement à l'opération A), on modifie le complexe polyol en associant dans le réacteur :
   ▪ 1,95 parties en poids de solution de catalyseur ;
   ▪ 0,49 parties en poids de débullant et
   ▪ 52,52 parties en poids de polyol à chaîne courte (VORANOL^{®} P400)
∘ On agite à 1300 trs/mn pendant 30 secondes. On obtient le complexe polyol.
∘ On ajoute dans ledit complexe polyol obtenu 45,04 parties en poids d'isocyanate selon une stoechiométrie OH/NCO=1, et on agite à 1300 trs/mn pendant 30 secondes. On obtient la solution de phase ;
o On coule ladite solution de phase obtenue sur une épaisseur de 1 mm sur la séquence « Réservoir » qui a dépassé le pot life mais qui n'a pas atteint le temps de démoulage ;
∘ On laisse la réaction de polymérisation se poursuivre pour constituer la séquence « Support-Barrière ».

### C)- Préparation de la solution de phase « Réservoir-Adhésive » de stoechiométrie OH/NCO = 1,5

∘ Parallèlement à l'opération B), on modifie le complexe polyol en associant dans le réacteur :
   ▪ 1,78 parties en poids de solution de catalyseur ;
   ▪ 0,44 partie en poids de débullant ;
   ▪ 8,89 parties en poids de préparation lipophile de caféine anhydre, dit l'actif et
   ▪ 72,35 parties en poids de polyol à chaîne longue (ETERNACOLL^{®} PH100).
∘ On agite le tout à 1300 trs/mn pendant 30 secondes. On obtient le complexe polyol chargé d'actif ;
∘ On ajoute dans ledit complexe polyol 16,54 parties en poids d'isocyanate selon une stoechiométrie OH/NCO = 1,5, et on agite à 1300 trs/mn pendant 30 secondes. On obtient la solution de phase chargée d'actif ;
∘ On coule sur une épaisseur de 1 mm, ladite solution de phase obtenue sur la séquence « Support-Barrière » qui a dépassé le pot life mais qui n'a pas atteint le temps de démoulage ;
∘ On laisse se continuer la polymérisation pour constituer la séquence «Réservoir-Adhésive »;
∘ On démoule le patch obtenu lorsque le temps de démoulage de ladite séquence « Réservoir-Adhésive » est atteint.

Le patch de bien-être et amincissant obtenu est très souple et collant sur la face de la séquence « Réservoir-Adhésive » chargée de caféine. Ledit patch présente une épaisseur de 3 mm. La matrice chargée en actifs dudit patch a la particularité de diffuser par volatilité le parfum se trouvant dans la séquence « Réservoir», de diffuser par voie transdermique la formulation de caféine se trouvant dans la séquence « Réservoir-Adhésive » lorsque ledit patch est appliqué sur la peau sur la face de la séquence « Réservoir-Adhésive ». La séquence « Support-Barrière » intercalée entre les deux séquences « Réservoir » joue un rôle de barrière empêchant à la fois la caféine de migrer vers la séquence « Réservoir » et le parfum de migrer vers la phase « Réservoir-Adhésive». Ledit patch est destiné au traitement pour l'amincissement chez l'homme.

### Exemple 5 : Plaquette auto-adhésive attractive de carpocapse en matrice ayant un profil « Réservoir/Support-Barrière/ Adhésive »

Cet exemple illustre l'élaboration d'une matrice monopolymérique apportant une fonction auto-adhésive d'une plaquette sur une seule face et un profil organisé en succession de trois séquences : une séquence réservoir chargée de phéromone, une séquence support barrière et une séquence adhésive.

On associe à l'isocyanate de l'exemple 1 un polyol à chaîne longue à base d'éther, plus précisément de type PTMEG de PM = 2000 et de fonctionnalité de 2. Ce polyol est produit par FORMOSA ASAHI SPANDEX et commercialisé sous la marque P2000 BX^{®}.

On dispose de :
- un allongeur de chaîne de PM = 149 et de fonctionnalité 3, le TRIETHANOLAMINE produit par BASF.
- un débullant BYK^{®} 066
- une préparation lipophile contenant une phéromone sexuelle produite par SHIN-ETSU.

On dispose du même matériel que celui utilisé dans l'exemple 1.

Mode opératoire : On procède selon un mode opératoire analogue à celui de l'exemple 1 et la solution de catalyseur est préparée comme dans l'exemple 1.

### A)- Préparation de la solution de phase « Réservoir » de stoechiométrie OH/NCO = 1

on introduit dans le réacteur dans cet ordre
   ▪ 1,91 parties en poids de solution de catalyseur ;
   ▪ 0,78 partie en poids d'allongeur ;
   ▪ 0,48 partie en poids de débullant ;
   ▪ 1,91 parties en poids de phéromone, dit l'actif et
   ▪ 79,48 parties en poids de polyol à chaîne longue de l'opération A).
∘ On agite à 1300 trs/mn pendant 30 secondes. On obtient le complexe polyol chargé de phéromone.
∘ On ajoute dans ledit complexe polyol obtenu 15,43 parties en poids d'isocyanate selon une stoechiométrie OH/NCO = 1, et on agite à 1300 trs/mn pendant 30 secondes. On obtient la solution de phase chargée d'actif ;
∘ On coule sur une épaisseur de 1 mm, ladite solution de phase obtenue pour constituer la séquence « Réservoir ».

### B)- Préparation de la solution de phase « Support-Barrière » de stoechiométrie OH/NCO = 1

o Parallèlement à l'opération A)-, on modifie le complexe polyol en associant dans le réacteur :
   ▪ 1,95 parties en poids de la solution de catalyseur ;
   ▪ 0,49 partie en poids de débullant et
   ▪ 59,65 parties en poids de polyol à chaîne longue
∘ On agite le tout à 1300 trs/mn pendant 30 secondes. On obtient le complexe polyol ;
∘ On ajoute dans ledit complexe polyol 37,92 parties en poids d'isocyanate selon une stoechiométrie OH/NCO = 1 et on agite à 1300 trs/mn pendant 30 secondes. On obtient la solution de phase ;
o On coule sur une épaisseur de 1 mm ladite solution de phase obtenue sur la séquence « Barrière » qui a dépassé le pot life mais qui n'a pas encore atteint le temps de démoulage et on laisse la réaction de polymérisation se faire pour constituer la séquence « Support-Barrière ».

### C)- Préparation de la solution de phase « Adhésive » de stoechiométrie OH/NCO = 1,5

∘ Parallèlement, on modifie le complexe polyol en associant dans le réacteur :
   ▪ 1,95 parties en poids de solution de catalyseur ;
   ▪ 0,49 parties en poids de débullant et
   ▪ 68,53 parties en poids de polyol à chaîne longue de l'opération A).
∘ On agite le tout à 1300 trs/mn pendant 30 secondes. On obtient le complexe polyol ;
∘ On ajoute dans ledit complexe polyol 29,04 parties en poids d'isocyanate selon une stoechiométrie OH/NCO = 1,5, et on agite à 1300 trs/mn pendant 30 secondes. On obtient la solution de phase ;
∘ On coule sur une épaisseur de 1 mm, ladite solution de phase obtenue sur la séquence « Support-Barrière » qui a dépassé le pot life mais qui n'a pas dépassé le temps de démoulage ;
∘ On laisse se continuer la polymérisation pour constituer la séquence « Adhésive » ;
∘ On démoule la plaquette ainsi obtenue lorsque le temps de démoulage de ladite séquence « adhésive » est atteint.

La plaquette obtenue d'une épaisseur de 3 mm, est souple et auto-adhésive sur la face de la séquence « Adhésive ». Ladite plaquette collée sur les branches des pommiers est destinée au traitement de ces derniers contre les carpocapses en diffusant la phéromone sexuelle en quantité contrôlée de manière à créer la confusion chez les mâles des carpocapses.

### Exemple 6 : Plaque parfumée avec « ventouses » auto-adhésive en matrice ayant un profil « Adhésive/ Support-Réservoir »

Cet exemple illustre l'élaboration d'une matrice monopolymérique active apportant une fonction auto-adhésive par des ventouses adhésives portées par la face discontinue de la séquence « Adhésive », selon un profil organisé en succession de deux séquences : une séquence adhésive et une séquence support réservoir chargée en actif. Les ventouses de 0,5 mm de haut permettent la circulation de l'air sous le support réservoir.

On associe à l'isocyanate de l'exemple 1 un polyol à chaîne longue, ALCUPOL^{®} C-3531.

On dispose d'un parfum commercialisé par CREATIONS & PARFUM sous la marque SHG^{®}61562

On dispose du même matériel que celui utilisé dans l'exemple 1 et d'un moule en PP constitué d'un disque circulaire ouvert de 1 cm d'épaisseur et de 15 cm de diamètre à l'intérieur duquel sont répartis cinq puits dont le diamètre est de 1,5 cm et 0,5 mm de profondeur.

Mode opératoire : On procède selon un mode opératoire analogue à celui de l'exemple 1 et la solution de catalyseur est préparée comme dans l'exemple 1.

### A)- Préparation de la solution de phase « Adhésive » de stoechiométrie OH/NCO = 1,5

∘ On introduit successivement dans le réacteur :
   ▪ 1,96 parties en poids de solution de catalyseur ;
   ▪ 91,50 parties en poids de polyol à chaîne longue.
∘ On agite le tout à 1300 trs/mn pendant 30 secondes. On obtient le complexe polyol ;
∘ On ajoute dans ledit complexe polyol obtenu 6,54 parties en poids d'isocyanate selon une stoechiométrie OH/NCO = 1,5, puis on agite à 1300 trs/mn pendant 30 secondes. On obtient la solution de phase ;
∘ On coule ladite solution de phase obtenue uniquement dans les puits du moule sur une profondeur de 5 mm ;
∘ On laisse la réaction de polymérisation se faire pour constituer la séquence « Adhésive ».

### B)- Préparation de la solution de phase « Réservoir-Support » de stoechiométrie OH/NCO = 1

Parallèlement à l'opération A), on modifie le complexe polyol en associant dans le réacteur :
▪ 1,64 parties en poids de solution de catalyseur ;
▪ 16,39 parties en poids de parfum, dit l'actif ;
▪ 74,03 parties en poids de polyol à chaîne longue de l'opération A).

∘ On agite à 1300 trs/mn pendant 30 secondes. On obtient le complexe polyol chargé d'actif ;
∘ On ajoute dans ledit complexe polyol obtenu 7,93 parties en poids d'isocyanate selon une stoechiométrie OH/NCO = 1, et on agite à 1300 trs/mn pendant 30 secondes. On obtient la solution de phase chargée d'actif ;
∘ On coule sur une épaisseur de 1 cm, ladite solution de phase obtenue sur la séquence « Adhésive » qui a dépassé le pot life mais qui n'a pas encore atteint le temps de démoulage ;
∘ On laisse se continuer la polymérisation pour constituer la séquence « Réservoir-Support » ;
∘ On démoule la plaque ainsi obtenue lorsque le temps de démoulage de ladite séquence « Réservoir-Adhésive » est atteint.

La plaque parfumante obtenue, d'une épaisseur de 1 cm, est très souple et présente des picots auto-adhésifs de 5 mm de longueur. Ladite plaque est destinée à parfumer une pièce par diffusion du parfum sur les deux faces de la séquence « Réservoir-Support ».

## Revendications

1. Procédé de préparation d'une matrice séquencée multifonctionnelle monopolymérique en polyuréthanne coulé à température ambiante, structurée concomitamment en séquence support ou support-barrière, séquence réservoir d'actif(s) et séquence adhésive, ladite matrice présentant une unicité ou continuité structurelle assurée par la copolymérisation à l'interface desdites séquences fonctionnelles successives de fonctionnalités différentes, lesdites séquences étant formées par des solutions de phase constituées par le mélange d'un complexe polyol liquide variable avec un isocyanate liquide, comprenant les étapes suivantes :
a) préparation du complexe polyol 1 qui consiste à mélanger la solution de catalyseur, les différents additifs et le cas échéant le(s) actif(s) correspondant à la fonction de la future séquence 1 et le(s) polyol(s) ;
b) préparation de la solution de phase 1 qui consiste à mélanger le complexe polyol 1 obtenu en a)- avec l'isocyanate selon la stoechiométrie correspondant à la fonction de la future séquence 1 ;
c) coulage de ladite solution de phase 1 dans le moule pour l'obtention de la séquence 1 en conformité de l'épaisseur souhaitée de ladite séquence 1 ;
d) préparation du complexe polyol 2 qui consiste à mélanger la solution de catalyseur, les différents additifs et le cas échéant le(s) actif(s) correspondant à la fonction de la future séquence 2 et le(s) polyol(s) ;
e) préparation de la solution de phase 2 qui consiste à mélanger le complexe polyol 2 obtenue en d)- avec l'isocyanate selon la stoechiométrie correspondant à la fonction de la future séquence 2 ;
f) coulage de ladite solution de phase 2 dans le moule pour l'obtention de la séquence 2, en conformité de l'épaisseur souhaitée de ladite séquence 2, sur la séquence 1 ayant dépassé le pot life mais n'ayant pas encore atteint le temps de démoulage, pour permettre une copolymérisation à l'interface des deux séquences ;
g) répéter les étapes a)- à f)- pour obtenir une matrice multifonctionnelle à « n » séquences, la dernière et/ou la première selon le profil choisi de la matrice étant une séquence au moins adhésive dans laquelle la solution de phase du complexe polyol avec l'isocyanate est de stoechiométrie supérieure à 1 ;
h) laisser s'achever la polymérisation de la dernière séquence jusqu'à son temps de démoulage et donc de l'ensemble de la matrice,
i) .démouler la matrice séquencée multifonctionnelle monopolymérique formée,
j) procéder à l'emballage de ladite matrice dans une poche scellée, étanche et imperméable

2. Procédé selon la revendication 1 ***caractérisé en* ce *que*** les principaux matériaux réactifs de polymérisation sont :
**a),** l'isocyanate choisi parmi les isocyanates ayant au minimum 2 fonctionnalités, de structure aromatique ou aliphatique ;
**b).** le(s) polyol(s) choisi(s) parmi ceux ayant au moins 2 fonctionnalités hydroxyles, de chaîne longue ou de chaîne courte, à base de polyesters, de polyéthers, de polythioéthers, de polyacétals, de polyesteramides, de polycarbonates, d'huiles végétales naturellement hydroxylées ou modifiées, ou le mélange de ceux-ci.

3. Procédé selon les revendications 1 et 2 ***caractérisé en ce que*** les additifs de formulation du complexe polyol sont choisis selon le type de fonction que va remplir chaque séquence et les caractéristiques souhaitées globalement du dispositif à former, que sont :
a). le catalyseur choisi parmi les solutions d'organométalliques, de préférence de bismuth, ou les solutions d'amines tertiaires ;
b). les débullants choisis parmi les polymères de polysiloxanes, les polymères de méthylalkylpolysiloxanes ou un mélange de ceux-ci ;
**c).** le plastifiant choisi parmi les solutions de benzoate d'ester, les alkyls phosphate ou les phtalates.
**d).** l'imperméabilisant choisi parmi les résines de faible poids moléculaire ou les charges inertes ;
**e).** les anti-UV choisis parmi ceux connus de l'homme du métier et les autres stabilisants choisis parmi les antioxydants phénoliques de type BHT (Butylhydroxytoluène) ou BHA (Butylhydroxyanisol), les antioxydants redox de type HALS (Hindered Amine Light Stabilizer) à motif pipéridine, les antioxydants de type phosphite, les antioxydants de type formamidine, les antioxydants de type benzotriazole, les antioxydants de type benzophénone ou un mélange de ceux-ci ;
f). les colorants de polyuréthannes.

4. Matrice susceptible d'être obtenu par le procédé selon l'une des revendications précédentes.

5. Matrice selon la revendication 4 ***caractérisée en ce qu***'elle présente des séquences fonctionnelles copolymérisées selon les profils suivants :
**A).** *Support* / *Réservoir* / *Adhésive ;*
**B).** *Réservoir 1* / *Support-Barrière* / *Réservoir 2-Adhésive ;*
**C).** *Support* / *Réservoir-Adhésive* ;
**D).** *Réservoir*/*Support*/*Barrière*/*Adhésive ;*
**E).** *Support*/*Réservoir 1*/*Réservoir 2*/*Adhésive* ;
**F).** *Support* / *Réservoir* 1 / *Réservoir 2-Adhésive.*

6. Utilisation d'une matrice selon la revendication 5 pour la mise en forme de dispositifs qui adhérent à la peau ou toute autre surface, tout en délivrant une ou plusieurs substances actives de manière contrôlée.

7. Utilisation d'une matrice selon la revendication 6 ***caractérisée en ce que*** les dispositifs sont des patchs, des emplâtres changeables collés sur support textile, des talonnettes actives et des coussinets actifs pour chaussures, des plaquettes à phéromone pour le contrôle des insectes ou des plaquettes auto-adhésives attractives insecticides.

## Patentansprüche

1. Verfahren zur Herstellung einer bei Umgebungstemperatur gegossenen multifunktionellen sequenzierten Monopolymer-Polyurethanmatrix, die gleichzeitig als Träger- oder Träger-Barriere-Sequenz, Wirkstoff-Behälter-Sequenz und Klebersequenz strukturiert ist, wobei die Matrix eine strukturelle Einheitlichkeit oder Kontinuität aufweist, die durch die Copolymerisation an der Grenzfläche der aufeinanderfolgenden funktionellen Sequenzen mit unterschiedlichen Funktionalitäten sichergestellt wird, wobei die Sequenzen durch Phasenlösungen gebildet werden, die aus dem Mischen eines variablen flüssigen Polyolkomplexes mit einem flüssigen Isocyanat bestehen, umfassend die folgenden Schritte:
a) Herstellung des Polyolkomplexes 1, der aus dem Mischen der Katalysatorlösung, der verschiedenen Zusatzstoffe und gegebenenfalls der Wirkstoffe, die der Funktion der künftigen Sequenz 1 entsprechen, und des Polyols/der Polyole besteht;
b) Herstellung der Phasenlösung 1, die aus dem Mischen des in a)-erhaltenen Polyolkomplexes 1 mit dem Isocyanat gemäß der Stöchiometrie, die der Funktion der künftigen Sequenz 1 entspricht, besteht;
c) Gießen der Phasenlösung 1 in die Form, um die Sequenz 1 in Übereinstimmung mit der gewünschten Dicke der Sequenz 1 zu erhalten;
d) Herstellung des Polyolkomplexes 2, der aus dem Mischen der Katalysatorlösung, der verschiedenen Zusatzstoffe und gegebenenfalls der Wirkstoffe, die der Funktion der künftigen Sequenz 2 entsprechen, und des Polyols/der Polyole besteht;
e) Herstellung der Phasenlösung 2, die aus dem Mischen des in d)- erhaltenen Polyolkomplexes 2 mit dem Isocyanat gemäß der Stöchiometrie, die der Funktion der künftigen Sequenz 2 entspricht, besteht;
f) Gießen der Phasenlösung 2 in die Form, um die Sequenz 2 in Übereinstimmung mit der gewünschten Dicke der Sequenz 2 auf der Sequenz 1 zu erhalten, die die Topfzeit überschritten, aber die Entformungszeit noch nicht erreicht hat, um eine Copolymerisation an der Grenzfläche der beiden Sequenzen zu ermöglichen;
g) Wiederholen der Schritte a)- bis f)-, um eine multifunktionelle Matrix mit "n" Sequenzen zu erhalten, wobei die letzte und/oder die erste der Matrix je nach dem gewählten Profil eine mindestens klebende Sequenz ist, in der die Phasenlösung des Polyolkomplexes mit dem Isocyanat eine Stöchiometrie größer als 1 aufweist;
h) Zulassen, dass die Polymerisation der letzten Sequenz bis zu ihrer Entformungszeit und damit der gesamten Matrix zum Abschluss kommt,
i) .Entformen der gebildeten multifunktionellen monopolymeren sequenzierten Matrix,
j) Durchführen der Verpackung der genannten Matrix in einem versiegelten, dichten und undurchlässigen Beutel

2. Verfahren nach Anspruch 1, ***dadurch gekennzeichnet, dass*** die wichtigsten reaktiven Polymerisationsmaterialien Folgende sind:
a). das Isocyanat, ausgewählt aus Isocyanaten mit mindestens 2 Funktionalitäten, mit aromatischer oder aliphatischer Struktur;
b). das/die Polyol(e), das/die aus denjenigen mit mindestens 2 Hydroxylfunktionalitäten, langkettig oder kurzkettig, auf Basis von Polyestern, Polyethern, Polythioethern, Polyacetalen, Polyesteramiden, Polycarbonaten, natürlich hydroxylierten oder modifizierten Pflanzenölen oder der Mischung davon ausgewählt ist/sind.

3. Verfahren nach den Ansprüchen 1 und 2, ***dadurch gekennzeichnet, dass*** die Zusatzstoffe zur Formulierung des Polyolkomplexes ausgewählt werden entsprechend der Art der Funktion, die jede Sequenz zu erfüllen hat, und den global gewünschten Eigenschaften der zu bildenden Vorrichtung, welche die Folgende sind:
a). Katalysator, der aus Lösungen von Organometallen, vorzugsweise Wismut, oder Lösungen von tertiären Aminen ausgewählt ist;
b). Blasenentfernungsmittel, die aus Polysiloxanpolymeren, Methylalkylpolysiloxanpolymeren oder einer Mischung davon ausgewählt sind;
c). Weichmacher, der aus Esterbenzoatlösungen, Alkylphosphaten oder Phthalaten ausgewählt ist.
d). Imprägniermittel, die unter den Harzen mit niedrigem Molekulargewicht oder den inerten Füllstoffen ausgewählt sind;
e). UV-Schutzmittel, die unter den Fachleuten bekannten UV-Schutzmitteln ausgewählt sind, und die anderen Stabilisatoren, die unter den phenolischen Antioxidantien vom Typ BHT (Butylhydroxytoluol) oder BHA (Butylhydroxyanisol), Redox-Antioxidantien vom Typ HALS (Hindered Amine Light Stabilizer) mit Piperidin-Einheit, Antioxidantien vom Phosphit-Typ, Antioxidantien vom Formamidin-Typ, Antioxidantien vom Benzotriazol-Typ, Antioxidantien vom Benzophenon-Typ oder eine Mischung davon ausgewählt sind;
f). Polyurethanfarbstoffe.

4. Matrix, die durch das Verfahren nach einem der vorhergehenden Ansprüche erhalten werden kann.

5. Matrix nach Anspruch 4, ***dadurch gekennzeichnet, dass*** sie funktionelle Sequenzen aufweist, die entsprechend den folgenden Profilen copolymerisiert sind:
A). *Träger* / *Behälter* / *Kleber;*
B). *Behälter 1* / *Träger-Barriere* / *Behälter 2-Kleber;*
C). *Träger* / *Behälter-Kleber;*
D). *Behälter* / *Träger-Barriere* / *Kleber;*
E). *Träger* / *Behälter 1* / *Behälter 2* / *Kleber;*
F). *Träger* / *Behälter 1* / *Behälter 2-Kleber.*

6. Verwendung einer Matrix nach Anspruch 5 zur Formgebung von Vorrichtungen, die an der Haut oder einer anderen Oberfläche kleben, während sie kontrolliert einen oder mehrere Wirkstoffe abgeben.

7. Verwendung einer Matrix nach Anspruch 6,
***dadurch gekennzeichnet, dass*** es sich bei den Vorrichtungen um Patches, austauschbare Pflaster, die auf textile Träger geklebt werden, aktive Fersenpolster und aktive Schuhpolster, Pheromonpads zur Insektenbekämpfung oder selbstklebende Insekten anziehende Insektizidpads handelt.

## Claims

1. A process for preparing a multifunctional sequenced cast polyurethane matrix at room temperature, concomitantly structured in support sequence or support-barrier sequence, active substance reservoir sequence and adhesive sequence, said matrix presenting a uniqueness or a structural continuity ensured by the state of copolymerisation at the interface of the successive functional sequences, said sequences being made by phase solution constituted by mixing a liquid polyol complex of a variable composition with a liquid isocyanate, comprising the following steps:
a) preparing the polyol complex 1, which consists of mixing the catalyst solution, the various additives, and, if appropriate, the active substance(s) corresponding to the function of the future sequence 1 and the polyol(s);
b) preparation of the phase solution 1, which consists of mixing the polyol complex 1 obtained in step a) with the isocyanate according to the stoichiometry corresponding to the function of the future sequence 1;
c) pouring of the said phase solution 1 in the mould to obtain the sequence 1 in conformity with the desired thickness of the said sequence 1;
d) preparation of the polyol complex 2, which consists of mixing the catalyst solution, the various additives, and, if appropriate, the active substance(s) corresponding to the function of the future sequence 2 and the polyol(s);
e) preparing of the phase solution 2, which consists of mixing the polyol complex 2 obtained in step d) with isocyanate according to the stoichiometry corresponding to the function of future sequence 2 ;
f) pouring the phase solution 2 in the mould to obtain the sequence 2; in conformity with the desired thickness of the said sequence 2, on the sequence 1 which has exceeded a pot life, but which has still not attained the demoulding time, in order to allow a copolymerisation at the interface of the two sequences;
g) repeat the steps a) to f) to obtain an "n" sequences multifunctional cast polyurethane matrix, the last and/or the first sequence according to the matrix's selected profile being a sequence at least adhesive in which the phase solution of the polyol complex with the isocyanate has a stoichiometry greater than 1;
h) let the polymerisation of the last sequence terminate up to its demoulding time and therefore up to the polymerisation of the whole of the matrix;
i) remove the formed multifunctional sequenced polyurethane matrix from the mould;
j) package the said matrix in a sealed, tight, and impermeable pocket.

2. The process according to claim 1 **characterized in that** the main reactive materials of polymerisation are:
a) the isocyanate selected from among the isocyanates with at least two functionalities of an aromatic or aliphatic structure;
b) the polyol(s) selected from among those with at least two hydroxyl functionalities, with a long chain or a short chain, based on polyesters, polyethers, polythioethers, polyacetals, polyesteramides, polycarbonates, naturally hydrolysed or modified vegetable oils, or the mixture thereof.

3. The process according to claim 1, **characterized in that** the polyol complex formulation additives are selected according to the type of function which each sequence will fulfil, and the globally desired characteristics of the device to be formed which are:
a) the catalyst selected from among the organometallic solutions, preferentially bismuth, or tertiary amine solutions;
b) the degazing agent selected from among the polymers of polysiloxanes, the polymers of methyl alkyl polysiloxanes or a mixture thereof;
c) the plasticizer selected from among the solutions of ester benzoate, the alkyl phosphates, or phtalates;
d) the impermeabilizer selected from among the resins of low molecular weight or inert loads;
e) the ultraviolet stabilizers selected from among those known by a person skilled in the art and the other stabilizers selected from among the phenolic antioxidants type BHT (Butylated hydroxytoluene) or BHA (Butylated hydroxyanisol), the redox antioxidants type HALS (Hindered Amine Light Stabilizer) with piperidine pattern, the phosphite antioxidants, the formamidine antioxidants, the benzotriazole antioxidants, the benzophenone antioxidants or a mixture thereof;
f) the dye of the polyurethanes.

4. Matrix capable of being obtained by the process according to any one of the preceding claims.

5. The matrix according to claim 4, **characterized in that** it includes copolymerised functional sequences according to the following profiles:
A). Support/Reservoir/Adhesive
B). Reservoir 1/Support-Barrier/Reservoir 2-Adhesive
C). Support/Reservoir-Adhesive
D). Reservoir/Support-Barrier/Adhesive
E). Support/Reservoir 1/Reservoir 2/Adhesive
F). Support/Reservoir 1/Reservoir 2-Adhesive.

6. Use of a matrix according to claim 5 to form different devices that adhere to the skin or any other surface, in order to deliver one or a plurality of substances in a controlled manner.

7. Use according to claim 6 **characterized in that** said devices are patches, replaceable plasters stuck to a textile support, active heel pads and ball pads for shoes, pheromone cards for controlling insects and self-adhesive insecticide attractant cards.
